(19) Europäisches Patentamt
European Patent Office
Office européen des brevets

(11) Veröffentlichungsnummer: **0 012 980**
**A1**

# (12) EUROPÄISCHE PATENTANMELDUNG

(21) Anmeldenummer: **79105239.2**

(22) Anmeldetag: **18.12.79**

(51) Int. Cl.$^3$: **C 07 C 143/64**
C 07 C 139/00

(30) Priorität: **23.12.78 DE 2855970**

(43) Veröffentlichungstag der Anmeldung:
**09.07.80 Patentblatt 80/14**

(84) Benannte Vertragsstaaten:
**CH DE FR GB**

(71) Anmelder: **HOECHST AKTIENGESELLSCHAFT**
**Zentrale Patentabteilung Postfach 80 03 20**
**D-6230 Frankfurt/Main 80(DE)**

(72) Erfinder: **Schläfer, Ludwig**
**Zum Gimbacher Hof 9A**
**D-6233 Kelkheim (Taunus)(DE)**

(72) Erfinder: **Hoyer, Ernst, Dr.**
**Eptingweg 3**
**D-6230 Frankfurt am Main 80(DE)**

## (54) Verfahren zur Herstellung von 2,5-Dialkoxy-anilin-4-sulfonsäuren.

(57) Ein Verfahren zur Herstellung einer 2,5-Dialkoxy-anilin-4-sulfonsäure durch Sulfierung eines 2,5-Dialkoxy-anilins oder deren N-Alkanoyl-Verbindung, wobei als Sulfierungsmittel Schwefelsäure oder Oleum verwendet wird; bei Einsatz der Alkanoylaminoverbindung als Ausgangsverbindung wird die als Zwischenprodukt hergestellte sulfierte Alkanoylaminoverbindung verseift. Die Sulfierungsreaktion wird bevorzugt mit der 3- bis 10-fach molaren Menge des Sulfierungsmittels und bevorzugt bei einer Temperatur zwischen 40 und 100°C durchgeführt.

2,5-Dialkoxy-anilin-4-sulfonsäuren dienen als Diazokomponenten zur Herstellung von Azofarbstoffen.

EP 0 012 980 A1

HOECHST AKTIENGESELLSCHAFT HOE 78/F 287 Dr.ST/sch

Verfahren zur Herstellung von 2,5-Dialkoxy-anilin-4-sulfonsäuren

Die Erfindung liegt auf dem Gebiet der Sulfierung von Anilinen zur Herstellung von wasserlöslichen Zwischenprodukten, insbesondere für Farbstoffe.

Niedere 2,5-Dialkoxy-anilin-4-sulfonsäuren sind in der Literatur als Diazokomponenten von Azofarbstoffen bekannt (s. Schweizerische Patentschrift 273 297), jedoch ist deren Herstellung nicht beschrieben.

Es ist weiterhin bekannt, Anisidine zu sulfieren. Hierbei tritt die Sulfogruppe in die ortho- und/oder in die para-Stellung zur Methoxygruppe ein (s. z.B. Houben-Weyl, Methoden der organischen Chemie, 4. Auflage (1955), Georg Thieme Verlag Stuttgart, Band IX, Seite 473). So entsteht aus 2-Methoxyanilin die 1-Methoxy-2-aminobenzol-4-sulfonsäure (s. auch BIOS Final Report 1153, 179 (1946)).

Desweiteren ist aus Houben-Weyl (loc. cit. Seite 472) bekannt, daß bei 2-Methoxyanilinen oder 2-Aminophenolen, sofern bei diesen die 4-Stellung besetzt ist, die Sulfogruppe in die ortho-Stellung zur Methoxy- oder Hydroxygruppe eintritt. So erhält man z.B. aus 4-Chlor-2-aminophenol die 4-Chlor-2-aminophenol-6-sulfonsäure.

Mit der vorliegenden Erfindung wurde ein Verfahren zur Herstellung von niederen Dialkoxyanilin-sulfonsäuren der allgemeinen Formel (1)

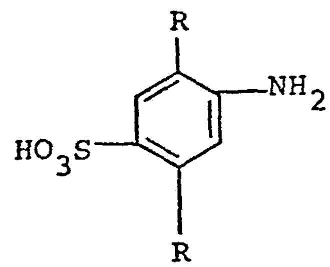

(1)

gefunden, in welcher R für eine niedere Alkoxygruppe, insbesondere für eine Methoxy- oder Äthoxygruppe, steht; die Angabe "niedere" steht hier wie im folgenden bevorzugt für eine Gruppe mit einem Alkylrest von 1 bis 4 C-Atomen. Mit diesem erfindungsgemäßen Verfahren werden die Verbindungen der Formel (1) ohne deren isomere Verbindungen erhalten. Das erfindungsgemäße Verfahren ist dadurch gekennzeichnet, daß man eine Verbindung der allgemeinen Formel (2)

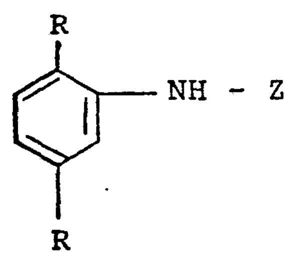

(2)

in welcher Z für ein Wasserstoffatom oder eine niedere Alkanoylgruppe, wie Acetylgruppe, steht und R die obengenannte Bedeutung hat, bei einer Temperatur zwischen -10°C und +120°C, vorzugsweise zwischen 20° und 100°C, insbesondere bevorzugt zwischen 40 und 70°C, mit einem Sulfierungsmittel aus der Gruppe Schwefelsäure und Oleum umsetzt.

Als Schwefelsäure wird eine 78 bis 100 gew.-%ige Schwefelsäure, als Oleum eine bis zu 30 Gew.-% Schwefeltrioxid enthaltende Schwefelsäure verwendet. Bevorzugt verwendet man als Sulfierungsmittel eine 85 bis 100%ige, insbesondere eine 96 bis 100%ige Schwefelsäure, oder eine Schwefelsäure mit einem Schwefeltrioxidgehalt von bis zu 10 Gew.-%. Das

Sulfierungsmittel wird in der 3- bis 10-fach molaren Menge, berechnet aus $SO_3$ und bezogen auf 1 Mol Ausgangsverbindung der Formel (2), eingesetzt; bevorzugt verwendet man das Sulfierungsmittel in der 4- bis 6-fach molaren Menge. Bei der Sulfierung kann das Sulfierungsmittel selbst als Reaktionsmedium dienen, jedoch können auch gegen Sulfierungsmittel inerte organische Lösungsmittel, wie beispielsweise Chlorbenzol oder Dichlorbenzol, Reaktionsmedium sein.

Die Sulfierungsreaktion der Verbindung der Formel (2) kann beispielsweise so ausgeführt werden, daß man die Verbindung der Formel (2) in fester oder in geschmolzener Form unter Rühren und Kühlen des Reaktionsmediums in das vorgelegte Sulfierungsmittel einträgt, wobei man vorteilhafterweise die Reaktionstemperatur zusätzlich durch Regelung der Zugabe der Ausgangsverbindung der Formel (2) kontrolliert und in dem gewünschten Reaktionstemperaturbereich hält, der bevorzugt zwischen 40 und 70°C liegt. Nach Zugabe der Ausgangsverbindung der Formel (2) rührt man noch einige Zeit, so beispielsweise 30 Minuten, nach, wobei man vorteilhaft die Temperatur bis auf 100°C erhöht. Nach Beendigung der Reaktion gibt man das Reaktionsgemisch in Wasser oder in ein Gemisch aus Wasser und Eis, wobei das Sulfierungsprodukt der allgemeinen Formel (1) als inneres Salz ausfällt. Nach Abkühlung des wäßrigen Zersetzungsgemisches wird das Produkt abgesaugt, mit Wasser gewaschen und getrocknet. Es wird in einer Ausbeute von 85 bis 98 % d. Th. erhalten.

Im Falle, daß man eine durch eine niedere Alkanoylgruppe geschützte 2,5-Dialkoxy-anilin-Verbindung einsetzt, d.h. Verbindungen der allgemeinen Formel (2), in welcher Z eine niedere Alkanoylgruppe bedeutet, erhält man die entsprechende 2,5-Dialkoxy-alkanoylamino-benzol-4-sulfonsäure, die in wäßrig-saurem Medium bei einem pH-Wert unterhalb von 2, vorzugsweise von 1, unter Abspaltung der Alkanoylgruppe verseift wird; vorzugsweise behandelt man die

2,5-Dialkoxy-alkanoylamino-benzol-4-sulfonsäure in der wäßrig sauren Lösung, die man nach Einbringung des Reaktionsgemisches in Wasser erhalten hat, bei einer Temperatur zwischen 60 und 120°C; aus dieser sauren Lösung fällt sodann die Aminosulfonsäure der allgemeinen Formel (1) als schwerlösliches inneres Salz aus.

In Kenntnis der oben erwähnten einschlägigen Literatur war es überraschend, daß die in 2- und 5-Stellung befindlichen beiden niederen Alkoxygruppen die in dem Benzolkern eintretende Sulfogruppe in die 4-Stellung zur Aminogruppe dirigieren und daß eine Sulfierung des 2,5-Dialkoxy-anilins in der 3- oder 6-Stellung nicht erfolgt. Es war nämlich zu erwarten, daß die 2-ständige Methoxygruppe zusätzlich die Sulfogruppe in deren ortho-Stellung dirigiert, ebenfalls daß die 5-ständige Methoxygruppe die Sulfogruppe in die andere ortho-Stellung dirigiert, so daß man ein Isomerengemisch aus den drei Isomeren 2,5-Dialkoxy-anilin-3-sulfonsäure, 2,5-Dialkoxy-anilin-4-sulfonsäure und 2,5-Dialkoxy-anilin-6-sulfonsäure erwarten mußte. Tatsächlich wird jedoch nur die 2,5-Dialkoxyanilin-4-sulfonsäure der allgemeinen Formel (1) erhalten. Isomere konnten nicht aufgefunden werden.

Die Konstitution der Verbindung der Formel (1) als Reaktionsprodukt des erfindungsgemäßen Verfahrens wurde durch C,H,N,O,S-Analyse gesichert, desweiteren durch NMR-Spektroskopie, die die erwartete para-Kupplung der beiden Protonen im Benzolkern, hingegen keine meta- oder ortho-Kupplung zeigt. Durch die übliche Analysenmethode, aromatische Amine zu diazotieren und mit einer Kupplungskomponente zu kuppeln und die erhaltene Azoverbindung dünnschichtchromatographisch zu untersuchen, wurde weiterhin gesichert, daß keine isomeren Verbindungen zur Verbindung der allgemeinen Formel (1) erhalten werden, da das Endprodukt nur einen einheitlichen Azofarbstoff bildet.

Die nachfolgenden Beispiele dienen zur Erläuterung der Erfindung.

Beispiel 1

153 Teile 2,5-Dimethoxyanilin werden unter guter Rührung und Kühlung innerhalb von 20 Minuten bei einer Temperatur zwischen 50 und 60°C in 500 Teile konzentrierter Schwefelsäure eingetragen; anschließend wird die Temperatur erhöht und 45 Minuten lang bei 90 bis 95°C weitergerührt. Man läßt das Reaktionsgemisch abkühlen und gibt es vorsichtig auf 1800 Teile Wasser. Der sich bildende Niederschlag wird bei 20°C abgesaugt, mit 100 Teilen Wasser gewaschen und bei 60°C unter reduziertem Druck getrocknet.

Man erhält die 2,5-Dimethoxyanilin-4-sulfonsäure in Form eines grauen Pulvers in einer Ausbeute von 85 % d. Th..

Beispiel 2

Zu 169 Teilen konzentrierter Schwefelsäure von einer Temperatur von 10°C werden innerhalb von 30 Minuten unter gutem Rühren 36,2 Teile 2,5 Diäthoxyanilin in geschmolzenem Zustand (50°C)hinzugegeben. Man hält die Temperatur des Reaktionsgemisches hierbei auf 40 bis 45°C und rührt eine Stunde lang nach. Anschließend wird das Reaktionsgemisch auf 150 Teile Eis gegeben, der Niederschlag abgesaugt und mit 60 Teilen Wasser gewaschen. Man erhält nach dem Trocknen die 2,5-Diäthoxyanilin-4-sulfonsäure in Form eines grauen Pulvers in einer Ausbeute von 96,5 % d.Th.. —
Im Abwasser wurden als diazotierbares Amin 3,5% des Ausgangsproduktes nachgewiesen.

Beispiel 3

306 Teile 2,5-Dimethoxy-anilin werden innerhalb von 30 Minuten bei einer Temperatur von maximal 65°C unter Rühren in 1150 Teile 100%ige Schwefelsäure eingetragen. Man rührt 30 Minuten lang bei dieser Temperatur nach, gibt sodann innerhalb von 60 Minuten 346 Teile Oleum mit einem Gehalt von 65% freiem Schwefeltrioxid bei einer Temperatur von maximal 70°C hinzu. Man rührt bei einer Temperatur von 70°C 1 bis 2 Stunden lang nach, läßt sodann abkühlen und gibt unter Rühren das Reaktionsgemisch auf ein Gemisch

von 200 Teilen Eis und 1200 Teilen Wasser. Die erhaltene Suspension wird weiterhin kalt gerührt, sodann abgesaugt, mit 200 Teilen Wasser gewaschen und getrocknet. Man erhält die 2,5-Dimethoxyanilin-4-sulfonsäure in einer Ausbeute von 98% d. Th..

Beispiel 4

200 Teile 2,5-Dimethoxyanilin werden in geschmolzener Form (40°C) innerhalb von 10 Minuten bei einer Temperatur von 70°C in 1250 Teile einer 78%igen Schwefelsäure eingetragen; man rührt eine Stunde lang bei einer Temperatur zwischen 90 und 100°C nach. Nach dem Abkühlen gibt man das Reaktionsgemisch auf 700 Teile Wasser, saugt das ausgefallene Produkt bei 20°C ab und wäscht es auf der Nutsche mit 200 Teilen Wasser. Die 2,5-Dimethoxyanilin-4-sulfonsäure wird in einer Ausbeute von 85% d. Th. erhalten.

Beispiel 5

390 Teile 2,5-Dimethoxy-acetanilid werden innerhalb von einer Stunde bei einer Temperatur von 40 bis 60°C und unter Rühren in 1500 Teile 15%iges Oleum eingetragen. Das Reaktionsgemisch wird im Verlaufe von 1 Stunde auf eine Temperatur von 70°C aufgeheizt und 2 Stunden lang bei dieser Temperatur gerührt. Anschließend gießt man es auf 4000 Teile Eiswasser, erwärmt das Gemisch eine Stunde lang bei einer Temperatur von 70 bis 80°C, kühlt es dann auf Raumtemperatur ab und filtriert die ausgefallene 2,5-Dimethoxyanilin-4-sulfonsäure. Nach dem Trocknen bestimmt sich die Ausbeute mit 93 % d.Th..

PATENTANSPRÜCHE:

1. Verfahren zur Herstellung von Verbindungen der allgemeinen Formel (1)

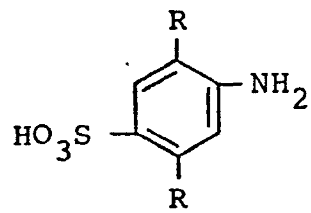

in welcher R für eine niedere Alkoxygruppe steht, dadurch gekennzeichnet, daß man eine Verbindung der allgemeinen Formel (2)

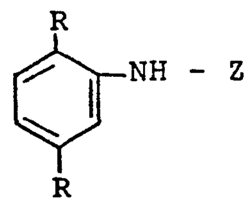 (2)

in welcher R die obengenannte Bedeutung hat und Z ein Wasserstoffatom oder eine niedere Alkanoylgruppe darstellt, mit einem Sulfierungsmittel aus der Gruppe Schwefelsäure und Oleum behandelt und, im Falle daß Z für eine niedere Alkanoylgruppe steht, die erhaltene 2,5-Dialkoxy-alkanoylamino-benzol-4-sulfonsäure zur Verbindung der Formel (1) verseift.

2. Verfahren nach Anspruch 1, dadurch gekennzeichnet, daß man als Sulfierungsmittel eine 78 bis 100%ige Schwefelsäure verwendet.

3. Verfahren nach Anspruch 1, daß man als Sulfierungsmittel eine Schwefelsäure mit einem Schwefeltrioxidgehalt von bis zu 30 Gew.-% verwendet.

4. Verfahren nach Anspruch 1, 2 oder 3, daß man die Schwefelsäure in der 3- bis 10-fach molaren Menge, berechnet auf $SO_3$ und bezogen auf Mol Ausgangsverbindung der allgemeinen Formel (2), einsetzt.

5. Verfahren nach einem der Ansprüche 1, 2, 3 oder 4, dadurch gekennzeichnet, daß man die Reaktion bei einer

Temperatur zwischen 40 und 100°C durchführt.

Europäisches Patentamt

# EUROPÄISCHER RECHERCHENBERICHT

0012980

Nummer der Anmeldung

EP 79 10 5239

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| | JOURNAL OF THE CHEMICAL SOCIETY, 1948, Part 1, London, GB, W.F. BEECH: "Preparation of certain nuclear-substituted 2-Aminophenol-sulphonic acids", Seiten 212-216<br>* Seite 213, Zeilen 18-20; Seite 215 "Direct sulphonation of 2-amino-4-methoxyphenol" *<br>-- | 1-5 | C 07 C 143/64<br>139/00 |
| | DE - A - 2 714 031 (BAYER AG)<br>* Ansprüche; Beispiel *<br>-- | 1-5 | RECHERCHIERTE SACHGEBIETE (Int. Cl.³)<br>C 07 C 143/64<br>139/00 |
| | CH - A - 273 298 (CIBA AG)<br>* Beispiel *<br>---- | 1-5 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
|---|---|---|
| Den Haag | 19-03-1980 | PAUWELS |